# FASCICULE DE BREVET EUROPEEN

(11) **EP 0 436 450 B1**
(45) Date de publication et mention de la délivrance du brevet: **16.03.1994**
(21) Numéro de dépôt: 90420556.4
(22) Date de dépôt: 20.12.1990
(51) Int. Cl.: A61K 9/50, C08J 3/12, B01J 13/02

(54) **Microsphères "core-shell" magnétisables à base d'organopolysiloxane réticulé, leur procédé de préparation et leur application en biologie**
Magnetisierbare Kern-Schale-Mikrokugeln, bestehend aus vernetztem Organopolysiloxan, Verfahren zu ihrer Herstellung und ihre Verwendung in der Biologie
Magnetisable core-shell microspheres based on crosslinked organopolysiloxane, their preparation process and their biological use

(30) Priorité: 27.12.1989 FR 8917232
(43) Date de publication de la demande: 10.07.1991
(73) Titulaire: SOCIETE PROLABO, 75011 Paris (FR)
(72) Inventeur: Charmot, Dominique, F-75019 Paris (FR)
(74) Mandataire: Ahner, Francis

(56) Documents cités:
- EP-A- 0 125 995
- EP-A- 0 319 828
- WO-A-87/02063
- DE-A- 3 002 477
- FR-A- 2 618 084
- FR-A- 2 624 873

## Description

La présente invention a pour objet des particules "core-shell" magnétisables à base d'organopolysiloxane réticulé, se présentant telles quelles ou en dispersion aqueuse, leur procédé de préparation et leur application en biologie.

La demanderesse a décrit dans sa demande de brevet français publiée sous le n° 2.624.873, des particules composites magnétisables à base d'organopolysiloxane réticulé, lesdites particules étant constituées d'une matrice dérivant de l'hydrosilylation d'un organopolysiloxane SiVi et d'un organohydrogrenopolysiloxane SiH et encapsulées dans ladite matrice de charges magnétisables revêtues d'un agent dispersant non hydrosoluble.

Dans ce type de produits les charges magnétisables sont revêtues d'un agent dispersant rendu non hydrosoluble. La présence de cet agent dispersant peut être un inconvénient en biologie car ledit agent peut migrer vers la surface des particules et entraîner des réactions secondaires.

La demanderesse a maintenant trouvé des microsphères composites dont le coeur est essentiellement constitué de charges magnétisables non recouvertes d'un agent surfactant hydrophobe.

Selon l'invention, il s'agit de microsphères magnétisables à base d'organopolysiloxane réticulé se présentant telles quelles ou en dispersion aqueuse, caractérisées en ce qu'elles sont formées :
- d'un coeur essentiellement constitué de charges magnétisables non revêtu d'un agent surfactant hydrophobe ou d'un agent dispersant présentant une taille généralement inférieure à 300 10⁻⁴ µm, de préférence de l'ordre de 50 à 120 10⁻⁴ µm
- et d'une écorce à base d'un organopolysiloxane réticulé dérivé de l'hydrosilylation

. d'au moins un organopolysiloxane (appelé SiVi) de formule I suivante :

   R'R₂ Si O (Si R R'' O)ₙ (Si R' R'' O)ₘ Si R₂ R' (I)

   formule dans laquelle :
. les symboles R sont identiques ou différents et représentent un radical alkyle en C₁ - C₄, phényle, trifluoro -3,3,3 propyle ;
. les symboles R' sont identiques ou différents et représentent R ou un radical vinyle, le nombre de radicaux vinyles étant de 2 au moins par macromolécule ;
. les symboles R'' sont identiques ou différents et représentent R ou un motif -r-X, où r représente un radical organique bivalent et X un groupe ionogène et/ou réactif non vinylique et non polycondensable choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
   halogénobenzyle, cyano, cyanato, 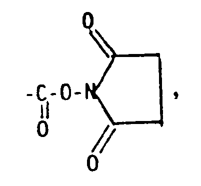 sulfate, et sulfonyle,
. au moins 60 % des radicaux représentés par les symboles R et R' sont des radicaux méthyles.
. les symboles n et m peuvent être nuls séparément, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000, de préférence de l'ordre de 5 à 500 par molécule d'organopolysiloxane SiVi et d'organohydrogénopolysiloxane SiH.
. avec au moins, un organohydrogénopolysiloxane (appelé SiH) contenant par molécule au moins trois atomes d'hydrogène liés chacun à un atome de silicium, présentant une viscosité de l'ordre de 5 à 1500 mPas à 25° C, de préférence entre 20 et 150 mPas à 25°C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
   halogénobenzyle, cyano, cyanato, 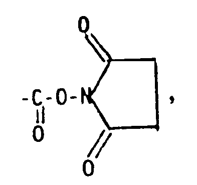 sulfate, et sulfonyle, liés à un atome de silicium ou à un atome de carbone d'un groupement hydrocarboné relié à la chaîne organohydrogénopolysiloxane par une liaison Si-C.

A titre d'exemple d'organopolysiloxane SiVi, on peut citer :
- les polymères de formule II

   (CH₂ = CH)R₂Si-O-(R R''Si-O)ₙ - SiR₂ (CH = CH₂) (II)

   où les symboles R et R '' ont la définition donnée ci-dessus, n ayant une valeur suffisante pour assurer une viscosité du polymère de 20 mPas à 30.000.000 mPas à 25°C
- et ceux de formule III

   R'₂ R Si-O (-R R'' Si-O)ₙ -[(CH₂ = CH)R'' Si-O-] ₘ -Si R R'₂ (III)

   où les symboles R, R'et R'' ont la signification donnée ci-dessus, n et m ayant une valeur suffisante pour assurer une viscosité du polymère de 20 mPas à 30.000.000 mPas à 25°C.

L'organohydrogénopolysiloxane SiH peut être linéaire, ramifié ou cyclique.

Parmi les organohydrogénopolysiloxanes SiH préferentiels on peut citer ceux de formule IV :

Y R₂ Si O (R R'' SiO)ₚ (YR SiO)_{q} SiR₂ Y (IV)

formule dans laquelle les symboles R sont identiques ou différents et ont la définition donnée ci-dessus, avec au moins 80 % de radicaux méthyles, le symbole Y représente R ou un atome d'hydrogène, le nombre d'atomes d'hydrogène étant de 3 au moins par molécule de polymère, le symbole R'' a la définition donnée ci-dessus, les symboles p et q étant tels que ledit polymère SiH présente une viscosité de l'ordre de 5 à 1500 mPas à 25°C, de préférence de l'ordre de 20 à 150 mPas à 25°C et un nombre de motifs ionogènes et/ou réactifs non vinyliques éventuels allant de 1 à 1000, de préférence de l'ordre de 5 à 500 par molécule d'organohydrogénopolysiloxane SiH et d'organopolysiloxane SiVi.

Parmi les radicaux organiques divalents pouvant constituer le radical r bivalent, on peut citer : les radicaux alkylènes linéaires ou ramifiés en C₁-C₁₈, éventuellement prolongés par 1 à 5 groupes bivalents éthylène amine, par 1 à 50 groupes oxyde d'alkylène en C₁-C₃, ou par un groupe 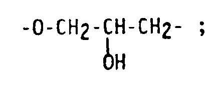
les radicaux polyoxyalkylènes contenant de 1 à 50 chaînons oxyalkylène en C₁-C₃.

A titre d'exemple de radicaux bivalents, on peut citer :
-CH₂- ; (̵CH₂)̵₂ ; (̵CH₂)̵₃ ; 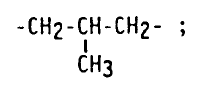
(̵CH₂)̵₁₀ ; (̵CH₂)̵₁₂ ;
(̵CH₂)̵₃NH-CH₂-CH₂- ; (̵CH₂)̵₃-OCH₂- ; (̵CH₂)̵₃(̵OCH₂-CH₂)̵₂₉ ;
(̵CH₂)̵₃[O-CH₂-CH(CH₃)] ₁₅ ;

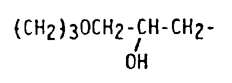

Les groupements réactifs ou ionogènes X constituant le symbole X, sont les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃, halogénobenzyle, cyano, cyanato, 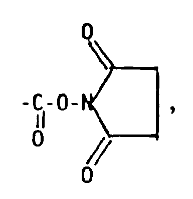
sulfate, et sulfonyle.

Les polymères SiVi et SiH ne portant pas de motif ionogène et/ou réactif non vinylique sont bien connus. Ils sont décrits par exemple dans les brevets américains n° 3 220 972 n° 3 344 111 et 3 436 366.

Les polymères SiVi et SiH portant des groupes ionogènes et/ou réactifs non vinyliques peuvent être préparés selon des méthodes bien connues.

Ainsi les polymères SiVi portant des groupes ionogènes et/ou réactifs non vinyliques peuvent être obtenus par :
- équilibrage d'un cyclotétrasiloxane et d'un cyclotétrasiloxane vinylé en présence d'un disiloxane fonctionnalisé
- équilibrage d'un cyclotétrasiloxane fonctionnalisé en présence d'un disiloxane divinylé
- équilibrage d'une huile polysiloxane fonctionnalisée en présence d'un divinylsiloxane et/ou d'un cyclotetrasiloxane
   ...

Les polymères SiH portant des groupes ionogènes et/ou réactifs non vinyliques peuvent être obtenus par exemple par :
- équilibrage d'un cyclotétrasiloxane et d'une huile polysiloxane à fonctions SiH internes en présence d'un disiloxane fonctionnalisé non réactif vis-à-vis des groupements SiH.
- équilibrage d'un cyclotétrasiloxane fonctionnalisé (non réactif vis-à-vis de groupements SiH) en présence d'un dihydrogénodisiloxane
- équilibrage d'une huile polysiloxane fonctionnalisée (non réactive vis-à-vis de groupements SiH) en présence d'un dihygénodisiloxane ou d'un polysiloxane à fonctions SiH internes
- ...

Pour une bonne réalisation de l'invention, l'écorce à base de polyorganosiloxane réticulé dérive de l'hydrosilylation d'au moins un organopolysiloxane SiVi et d'au moins un organohydrogénopolysiloxane SiH, avec des quantités relatives des deux types de polymère telles que le rapport en nombre des "groupes SiH" (atome d'hydrogène lié à un atome de silicium) sur les "groupes SiVi" (groupe vinyle lié à un atome de silicium) soit compris entre 0,75/1 et 4/1, de préférence entre 0,75/1 et 1,5/1.

Parmi les matériaux pouvant constituer les charges magnétisables formant le coeur des particules de l'invention, on peut citer la magnétite, l'hématite, le dioxyde de chrome, les ferrites telles que les ferrites de manganèse, nickel, manganèse-zinc ..., les alliages de cobalt, nickel, gadolinium, samarium-cobalt ... Les matériaux préférentiels sont la magnétite et l'hématite.

A côté des charges magnétisables peuvent être présentes des charges présentant un spectre de fluorescence telles que oxyde ou oxysulfure d'yttrium activé à l'europium, borate de gadolinium - cerium - terbium, aluminate de cerium-terbium, aluminate de magnésium - baryum dopé à l'europium divalent...

La quantité de charges magnétisables formant le coeur correspond à environ 0,5 à 98 % (dont 0,01 % à 0,5 % de charges fluorescentes éventuelles) en poids desdites charges par rapport au poids de microsphères et de préférence de 5 à 80 % en poids.

Les microsphères magnétisables faisant l'objet de l'invention sont parfaitement sphériques ; elles peuvent être de taille uniforme ou présenter une granulométrie étalée ; leur diamètre peut être de l'ordre de 0,05 à 3 microns et généralement de l'ordre de 0,2 à 2 µm.

Elles peuvent se présenter telles quelles ou en dispersion dans l'eau ; la quantité de microsphères magnétisables à l'état dispersé dans l'eau peut correspondre à environ 10 à 70 % en poids par rapport au poids total de dispersion et généralement de l'ordre de 15 à 50 % en poids.

Les microsphères "core-shell" magnétisables faisant l'objet de l'invention peuvent être préparées selon un procédé consistant à :
- disperser dans un solvant organique non miscible à l'eau une suspension aqueuse de charges magnétisables non revêtues d'agent dispersant, charges présentant une taille généralement inférieure à 300 10⁻⁴ µm, de préférence de l'ordre de 50 10⁻⁴ à 120 10⁻⁴ µm, ainsi qu'un catalyseur d'hydrosilylation
- à dissoudre dans la phase organique de la dispersion obtenue, un mélange

. d'au moins un organopolysiloxane (appelé SiVi) de formule I
. et d'au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25°C, de préférence entre 20 et 150 mPas à 25°C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
   halogénobenzyle, cyano, cyanato, 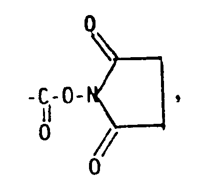 sulfate, et sulfonyle, liés à un atome de silicium ou à un atome de carbone d'un groupement hydrocarboné relié à la chaîne organohydrogénopolysiloxane par une liaison Si-C.

- à réticuler le mélange de polymères SiVi et SiH
- à éliminer l'eau
- à séparer les microsphères magnétisables
- et éventuellement à redisperser lesdites microsphères dans de l'eau

Le solvant organique mis en oeuvre à l'étape de dispersion est un solvant des polymères SiVi et SiH. A titre d'exemple on peut citer le cyclohexane, le chlorure de methylène, le benzène, l'hexane, l'octane, le toluène, le tetrachlorure de carbone, l'octane, les esters de diacides gras.

L'opération de dispersion est réalisée en une ou plusieurs étapes à une température de l'ordre de 20 à 60°C, à l'aide d'un système d'agitation énergique tel que moulin à colloïdes, pompes à haute pression, agitateur à vibrations, appareil à ultra-sons ...

La suspension aqueuse de charges magnétisables peut être obtenue par mise en suspension de charges broyées ; toutefois une forme préférentielle de suspension est un sol aqueux de charges magnétisables obtenu par tout procédé connu comme par exemple celui décrit dans le brevet US n° 3.480.555.

Le type de matériau pouvant constituer les charges a déjà été mentionné ci-dessus. D'autres charges peuvent être présentes à côté des charges magnétisables, comme par exemple les charges fluorescentes citées ci-dessus.

La concentration de charges magnétisables dans la suspension aqueuse peut être de l'ordre de 0,5 à 50 % en poids, généralement de l'ordre de 5 à 20 % en poids. La quantité de charges mise en oeuvre est telle que le rapport pondéral charges magnétisables/mélange de polymères SiVi et Si H soit de l'ordre de 0,005 à 50.

La quantité de solvant organique mise en oeuvre est telle que le rapport pondéral phase aqueuse/phase organique soit de l'ordre de 0,005 à 2.

Un agent tensio-actif est mis en oeuvre pour réaliser l'opération de dispersion. Celui-ci peut être choisi parmi ceux permettant d'obtenir des émulsions eau dans l'huile (de HLB généralement inférieur à 10, de préférence inférieur à 5) tels que les agents non-ioniques du type esters d'acides gras du sorbitol, mono et trioléates de sorbitan, copolymères séquencés oxyde d'éthylène/oxyde de propylène, les alkylphénols ethoxylés contenant moins de 10 motifs ethoxylés, les polycondensats d'acides gras, les copolymères séquencés organosiloxane - oxyde d'éthylène - oxyde de propylène ; les agents anioniques tels que les dialkylsulfosuccinates ; les agents cationiques tels que le bromure de cetylammonium, les copolycondensats polyethylèneimine - polyester.

On peut utiliser comme catalyseur de silylation des composés d'un métal du groupe du platine, en particulier leurs sels et complexes notamment l'acide chloroplatinique et les complexes de platine-oléfine comme décrit dans les brevets US-A 3 159 601 et 3 159 662, les produits de réaction des dérivés du platine avec des alcools, des aldéhydes et des éthers décrits dans le brevet US-A-3 220 972, les catalyseurs platine-vinylsiloxane décrits dans les brevets français FR-A 1 313 846 et son addition 88 676 et le brevet français FR-A 1 480 409 ainsi que les complexes décrits dans les brevets US-A 3 715 334, 3 775 452 et 3 814 730, ainsi qu'un catalyseur au rhodium tel que décrit dans les brevets US-A 3 296 291 et 3 928 629.

Les métaux préférés du groupe du platine sont le platine et le rhodium ; le ruthénium bien que moins actif mais moins cher, est également utilisable.

La quantité de catalyseur mise en oeuvre est généralement de l'ordre de 5 à 100 ppm, de préférence de 10 à 60 ppm, de catalyseur calculée en poids de métal par rapport au poids total des polymères SiVi et SiH.

Des exemples de polymères SiVi et SiH pouvant être utilisés ont déjà été cités ci-dessus. Pour une bonne réalisation de l'invention, les quantités relatives des deux types de polymère sont telles que le rapport en nombre des "groupes SiH" (atome d'hydrogène lié à un atome de silicium) sur les groupes SiVi (groupe vinyle lié à un atome de silicium) soit compris entre 0,75/1 et 4/1, de préférence entre 0,75/1 et 1,5/1.

L'opération de réticulation peut être réalisée à une température de l'ordre de 20 à 90°C, de préférence de l'ordre de 50 à 70°C. Cette opération dure généralement 2 à 24 heures environ.

L'eau est ensuite éliminée par distillation par exemple.

Après refroidissement les microsphères magnétisables peuvent être séparées du milieu organique par tout moyen connu, notamment par aimantation.

Si désiré lesdites microsphères magnétisables peuvent être redispersées dans de l'eau déionisée jusqu'à obtenir un taux d'extrait sec de l'ordre de 10 à 70 % en poids, de préférence de l'ordre de 15 à 50 % en poids. Cette opération est réalisée en présence d'au moins un agent tensio-actif permettant d'obtenir des emulsions huile dans l'eau (de HLB généralement supérieur à 10, de préférence supérieur à 15) tel que alkylsulfates, alkylsulfonates...

Les microsphères magnétisables faisant l'objet de l'invention sont notamment intéressantes en biologie.

Elles peuvent être utilisées par exemple comme supports actifs :
. d'anticorps ou d'antigènes pour les tests de diagnostics, les séparations par affinité des composés biologiques ; la fixation des molécules biologiques peut, si nécessaire, être réalisée par des méthodes de couplage bien connues faisant intervenir des agents de couplage (glutaraldéhyde, carbodiimide hydrosoluble), ou bien consistant à activer les fonctions éventuelles du polyorganosiloxane (par exemple par diazotation, par action du bromure de cyanogène, de l'hydrazine ...) et à faire réagir la molécule à fixer, etc ...
. de systèmes enzymatiques pour réactions biologiques
. de fixation de cultures cellulaires
. de médicaments ou de substances de révélation pour guider ceux-ci ou celles-ci in vitro ou in vivo vers le point de traitement choisi
. de molécules chimiques permettant une croissance de ces molécules par enchaînement rapide de réactions particulières comme la synthèse peptidique
. de groupements chimiques catalyseurs de réaction
. de groupements chimiques pour la séparation ou l'extraction de métaux ou d'isomères optiques.

Lesdites microsphères peuvent également être utilisées comme charges de renforcement pour élastomères ou pour la préparation de dispersions organiques utilisées dans les circuits hydrauliques de freins et d'amortisseurs.

Lorsqu'elles renferment en outre des charges luminescentes, elles peuvent être mises en oeuvre comme marqueur cellulaire ou comme agent de contraste en imagerie médicale.

La suspension aqueuse non surfactée d'oxyde de fer magnétique mise en oeuvre dans les exemples ci-après est préparée de la manière suivante :
175 g de Fe(N03)3,9H20 et 75 g de Fe(SO4),7H20 sont dissous dans 250 g d'eau permutée et 55 g d'acide nitrique concentré ; sous agitation rapide on ajoute 250 g d'une solution aqueuse à 20 % d'ammoniaque. Après décantation et élimination de la solution surnageante, le précipité est lavé une fois par de l'eau. Le milieu est ensuite ajusté à pH 0,5 par 35 g d'acide perchlorique, et le précipité filtré ; cette opération est répétée 3 fois, après quoi l'oxyde est remis en suspension dans de l'eau et ultrafiltré par de l'eau permutée. La suspension ainsi obtenue a une teneur en solides de 26,5 % pour un pH de 1,2. Le rendement exprimé en Fe304 est de 57 %. L'examen en microscopie electronique à transmission indique des tailles de particule d'oxyde de fer comprises entre 50 10⁻⁴ et 200 10⁻⁴ µm.

### EXEMPLE 1 :

Préparation de microsphères magnétisables "core-shell" à écorce en polymethylsiloxane réticulé

2 g de la suspension d'oxyde de fer préparée ci-dessus en mélange avec 0,4 g d'une solution aqueuse d'acide chloroplatinique à 0,25 % en poids, sont dispersés dans un mélange constitué de 50 g de Solvesso 200® (coupe polyaromatique petrolière fournie par Esso (France)) et 0,1 g de SPAN 80® (monooléate de sorbitan commercialisé par ICI (UK)), à l'aide d'un homogeneiseur ultrasonique. Cette émulsion inverse est chargée dans un réacteur de verre de 50 ml thermorégulé, muni d'une agitation mécanique et d'un condenseur. La température est amenée à 50° C et on introduit pendant une durée de 1 heure 5,62 g d'un mélange constitué de :
* 4,07 g d'une huile organosiliciée divinylée (dénommée huile SI-vinylA) de fomule I
   R''R'RSiO-(SiRR'''O)ₙ-(SiR'''R'O)ₘ-SiRR'R'',
   où R=R'=R'''=CH3,R''=-CH:CH2
   avec n+m=142
* 0,55 g d'une huile organosiliciée divinylée (dénommée huile Si-vinyl B) de formule I
   R''R'RSiO-(SiRR'''O)ₙ-(SiR'''R'O)ₘ-SiRR'R'',
   où : R=R'=R'''=-CH3,R''=-CH:CH2
   n+m=24
* 1 g d'une huile organosiliciée hydrosilylée (dénommée huile Si-H C) de formule IV R'₃SiO-(SiRR''O)ₒ-(SiRR'''O)ₚ-(SiYRO)_{q}-SiR'₃,
   où R=R'=R''=R'''=-CH3,Y=H
   avec, o+p=16 et q=42

Le milieu est agité pendant 15h, puis l'eau présente dans l'émulsion est ensuite éliminée par distillation azéotropique. Les microsphères sont récupérées par aimantation et lavées par de l'acetone et redispersées dans l'eau en présence de Cemulsol NP 30® (nonyl-phénol-ethoxylé à 30 molécules d'oxyde d'ethylene, commercialisé par SFOS (France)) à la concentration de 1 g/l, pour former un latex magnétique à un extrait sec de 10 %. Le rendement d'hydrosylilation est proche de 100 % (exprimé en poids de polydimethylsiloxane réticulé à la surface des particules).

La teneur en oxyde de fer dans les particules est de 8 % en poids, estimée par dosage du fer en absorption atomique. Les tailles de particules sont comprises entre 0,1 µm et 0,5 µm (mesurées en microscopie électronique à transmission).

### EXEMPLE 2 :

L'exemple 1 est répété à la différence que le SPAN 80® est remplacé par l'Hypermer LP8® (agent dispersant commercialisé par ICI (UK)). Le mélange d'huile Si-vinyl et d'huile Si-H est composé de 4,07 g d'huile Si-vinyl A et 1 g d'huile Si-H C.

Le rendement d'hydrosylilation est proche de 100 % (exprimé en poids de polymethylsiloxane réticulé à la surface des particules). La teneur en oxyde de fer dans les particules est de 9 %.

### EXEMPLE 3 :

L'exemple 1 est répété à la différence que le Solvesso 200® est remplacé par l'octane. Le mélange d'huile Si-vinyl et d'huile Si-H est composé de 4,54 g d'huile Si-vinyl A et 0,5 g d'huile Si-H C. Le rendement d'hydrosylilation est de 28 % (exprimé en poids de polydimethylsiloxane réticulé à la surface des particules). La teneur en oxyde de fer dans les particules est de 27 %.

### EXEMPLE 4 :

Préparation de microsphères magnétisables "core-shell" à écorce en polydimethylsiloxane réticulé fonctionnalisé époxy

L'exemple 1 est répété à la différence que le mélange d'huile Si-vinyl et d'huile SI-H est composé de
* 2 g d'huile Si-vinyl A
* 0,5 g d'huile organosiliciée hydrosilylée et époxydée de formule R'₃SiO-(SiRR''O)ₒ)-(SiRR'''O)ₚ-(SiYRO)_{q}-SiR'3
   où R=R'=R''=-CH3, Y=H, R'''=ether de glycidyl
   avec o=6, p=6, q=6.

Le rendement d'hydrosilylation est de 31 % (exprimé en poids de polydimethylsiloxane époxydé réticulé à la surface des particules). La teneur en oxyde de fer dans les particules est de 36 %.

### EXEMPLE 5 :

Préparation de microsphères magnétisables "core-shell" à écorce en polydimethylsiloxane réticulé fonctionnalisé amine

On reprend 2 g de microsphères préparées à l'exemple 4 que l'on redisperse dans 50 g de toluene. La dispersion est ensuite chargée dans le réacteur décrit dans l'exemple 1. La température est amenée à 100° C ; on verse alors au goutte à goutte 1,1 g d'une diamine alkoxylée de formule H₂N-CH(CH₃)CH₂-[O-CH(CH₃)CH₂]ₐ-[O-CH2CH2]_{b}-[O-CH(CH3)CH2]_{c}-NH2 (Jeffamine® commercialisée par Texaco (USA)). Le milieu réactionnel est laissé à cette température pendant 15 heures. Après refroidissement, l'excès d'amine est éliminé par décantation magnétique. Les microsphères aminées sont ensuite redispersées dans l'eau pour obtenir un latex magnétisable.

### EXEMPLE 6 :

Dans cet exemple on substitue la suspension aqueuse non surfactée d'oxyde de fer par une suspension d'oxyde de fer surfactée préparée selon le procédé revendiqué dans le brevet US 4094804 ; il s'agit d'oxyde de fer précipité en présence d'acide oleique, que l'on repeptise en milieu aqueux par ajout d'émulsifiant anionique (dioctylsulfosuccinate, Aerosol OT® commercialisé par American Cyanamid). La synthèse est poursuivie comme indiqué dans l'exemple 1 : dans ce cas on n'obtient pas de microsphères magnétisables ; en effet l'oxyde de fer a diffusé progressivement de la phase aqueuse vers la phase organique.

## Revendications (Revendications pour l'(les) Etat(s) contractant(s) suivant(s): AT, BE, CH, DE, DK, FR, GB, GR, IT, LI, LU, NL, SE)

1. Microsphères magnétisables à base d'organopolysiloxane réticulé se présentant telles quelles ou en dispersion aqueuse, caractérisées en ce qu'elles sont formées :
- d'un coeur essentiellement constitué de charges magnétisables non revêtu d'un agent surfactant hydrophobe ou d'un agent dispersant présentant une taille généralement inférieure à 300 10⁻⁴ µm
- et d'une écorce à base d'un organopolysiloxane réticulé dérivé de l'hydrosilylation
. d'au moins un organopolysiloxane (appelé SiVi) de formule I suivante :
R'R₂ Si O (Si R R'' O)ₙ (Si R' R'' O)ₘ Si R₂ R' (I)
formule dans laquelle :
. les symboles R sont identiques ou différents et représentent un radical alkyle en C₁ - C₄, phényle, trifluoro -3,3,3 propyle ;
. les symboles R' sont identiques ou différents et représentent R ou un radical vinyle, le nombre de radicaux vinyles étant de 2 au moins par macromolécule ;
. les symboles R'' sont identiques ou différents et représentent R ou un motif -r-X, où r représente un radical organique bivalent et X un groupe ionogène et/ou réactif non vinylique et non polycondensable choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 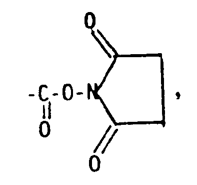 sulfate, et sulfonyle,
. au moins 60 % des radicaux représentés par les symboles R et R' sont des radicaux méthyles.
. les symboles n et m peuvent être nuls séparément, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000 par molécule d'organopolysiloxane SiVi et d'organohydrogénopolysiloxane SiH.
. avec au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25°C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 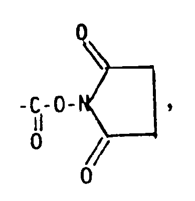 sulfate, et sulfonyle, liés à un atome de silicium ou à un atome de carbone d'un groupement hydrocarboné relié à la chaîne organohydrogénopolysiloxane par une liaison Si-C.

2. Microsphères selon la revendication 1 caractérisées en ce que les charges magnétisables présentent une taille de 50 à 120 10⁻⁴ µm.

3. Microsphères selon la revendication 1 ou 2 caractérisées en ce que la quantité de charges magnétisables constituant le coeur représente 5 à 98 % du poids des microsphères.

4. Microsphères selon l'une quelconque des revendications 1 à 3 caractérisées en ce que l'organohydrogenopolysiloxane SiH a pour formule
Y R₂ Si O (R R'' SiO)ₚ (YR SiO)_{q} SiR₂ Y (IV)
formule dans laquelle les symboles R sont identiques ou différents et ont la définition donnée ci-dessus, avec au moins 80 % de radicaux méthyles, le symbole Y représente R ou un atome d'hydrogène, le nombre d'atomes d'hydrogène étant de 3 au moins par molécule de polymère, le symbole R'' a la définition donnée à la revendication 1, les symboles p et q étant tels que ledit polymère SiH présente une viscosité de l'ordre de 5 à 1500 mPas à 25°C et un nombre de motifs ionogènes et/ou réactifs non vinyliques éventuels allant de 1 à 1000, par molécule d'organohydrogénopolysiloxane SiH et d'organopolysiloxane SiVi.

5. Microsphères selon l'une quelconque des revendications 1 à 4 caractérisées en ce que les quantités relatives d'organopolysiloxane SiVi et d'organohydrogenosiloxane SiH correspondent à un rapport en nombre des "groupes SiH" sur les "groupes SiVi" compris entre 0,75/1 et 4/1.

6. Procédé de préparation de microsphères magnétisables à base d'organopolysiloxane réticulé consistant à :
- disperser dans un solvant organique non miscible à l'eau une suspension aqueuse de charges magnétisables non revêtues d'agent dispersant, charges présentant une taille généralement inférieure à 300 10⁻⁴ µm, ainsi qu'un catalyseur d'hydrosilylation
- à dissoudre dans la phase organique de la dispersion obtenue, un mélange
. d'au moins un organopolysiloxane (appelé SiVi) de formule I suivante :
R'R₂ Si O (Si R R'' O)ₙ (Si R' R'' O)ₘ Si R₂ R' (I)
formule dans laquelle :
. les symboles R sont identiques ou différents et représentent un radical alkyle en C₁ - C₄, phényle, trifluoro -3,3,3 propyle ;
. les symboles R' sont identiques ou différents et représentent R ou un radical vinyle, le nombre de radicaux vinyles étant de 2 au moins par macromolécule ;
. les symboles R'' sont identiques ou différents et représentent R ou un motif -r-X, où r représente un radical organique bivalent et X un groupe ionogène et/ou réactif non vinylique et non polycondensable choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 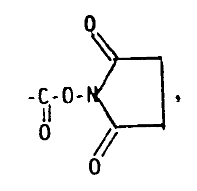 sulfate, et sulfonyle,
. au moins 60 % des radicaux représentés par les symboles R et R' sont des radicaux méthyles.
. les symboles n et m peuvent être nuls séparément, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000 par molécule d'organopolysiloxane SiVi et d'organohydrogénopolysiloxane SiH.
. et d'au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25° C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 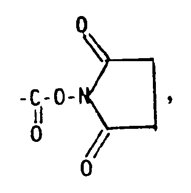 sulfate, et sulfonyle, liés à un atome de silicium ou à un atome de carbone d'un groupement hydrocarboné relié à la chaîne organohydrogénopolysiloxane par une liaison Si-C.
- à réticuler le mélange de polymères SiVi et SiH
- à éliminer l'eau
- à séparer les microsphères magnétisables
- et éventuellement à redisperser lesdites microsphères dans de l'eau

7. Procédé selon la revendication 6 caractérisé en ce que les charges magnétisables ont une taille de l'ordre de 50 à 120 10⁻⁴ µm.

8. Procédé selon la revendication 6 ou 7 caractérisé en ce que la concentration de charges magnétisables dans la suspension aqueuse est de l'ordre de 0,5 à 50 % en poids et en ce que la quantité de charges mise en oeuvre est telle que le rapport pondéral charges magnétisables/mélange de polymères SiVi et SiH soit de l'ordre de 0,005 à 50.

9. Procédé selon l'une quelconque des revendications 6 à 8 caractérisé en ce que la quantité de solvant organique mise en oeuvre est telle que le rapport pondéral phase aqueuse/phase organique soit de l'ordre de 0,005 à 2.

10. Procédé selon l'une quelconque des revendications 6 à 9 caractérisé en ce que l'organohydrogenopolysiloxane a pour formule
Y R₂ Si O (R R'' SiO)ₚ (YR SiO)_{q} SiR₂ Y (IV)
formule dans laquelle les symboles R sont identiques ou différents et ont la définition donnée ci-dessus, avec au moins 80 % de radicaux méthyles, le symbole Y représente R ou un atome d'hydrogène, le nombre d'atomes d'hydrogène étant de 3 au moins par molécule de polymère, le symbole R'' a la définition donnée à la revendication 6, les symboles p et q étant tels que ledit polymère SiH présente une viscosité de l'ordre de 5 à 1500 mPas à 25°C et un nombre de motifs ionogènes et/ou réactifs non vinyliques éventuels allant de 1 à 1000, par molécule d'organohydrogénopolysiloxane SiH et d'organopolysiloxane SiVi.

11. Procédé selon l'une quelconque des revendications 6 à 10 caractérisé en ce que les quantités relatives d'organopolysiloxane SiVi et d'organohydrogenosiloxane SiH correspondent à un rapport en nombre des "groupes SiH" sur les "groupes SiVi" compris entre 0,75/1 et 4/1.

12. Utilisation des microsphères magnétisables faisant l'objet de l'une quelconque des revendications 1 à 5 ou obtenues selon le procédé faisant l'objet de l'une quelconque des revendications 6 à 11 comme supports actifs en biologie, à condition que les méthodes de traitement thérapeutique telles que définies à l'articles 52(4) CBE soient exclues.

## Revendications (Revendications pour l'(les) Etat(s) contractant(s) suivant(s): ES)

1. Procédé de préparation de microsphères magnétisables à base d'organopolysiloxane réticulé se présentant telles quelles ou en dispersion aqueuse, caractérisées en ce qu'elles sont formées :
- d'un coeur essentiellement constitué de charges magnétisables non revêtu d'un agent surfactant hydrophobe ou d'un agent dispersant présentant une taille généralement inférieure à 300 10⁻⁴ µm
- et d'une écorce à base d'un organopolysiloxane réticulé dérivé de l'hydrosilylation
. d'au moins un organopolysiloxane (appelé SiVi) de formule I suivante :
R'R₂ Si O (Si R R'' O)ₙ (Si R' R'' O)ₘ Si R₂ R' (I)
formule dans laquelle :
. les symboles R sont identiques ou différents et représentent un radical alkyle en C₁ - C₄, phényle, trifluoro -3,3,3 propyle ;
. les symboles R' sont identiques ou différents et représentent R ou un radical vinyle, le nombre de radicaux vinyles étant de 2 au moins par macromolécule ;
. les symboles R'' sont identiques ou différents et représentent R ou un motif -r-X, où r représente un radical organique bivalent et X un groupe ionogène et/ou réactif non vinylique et non polycondensable choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 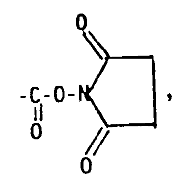 sulfate, et sulfonyle,
. au moins 60 % des radicaux représentés par les symboles R et R' sont des radicaux méthyles.
. les symboles n et m peuvent être nuls séparément, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000 par molécule d'organopolysiloxane SiVi et d'organohydrogénopolysiloxane SiH.
. avec au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25°C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 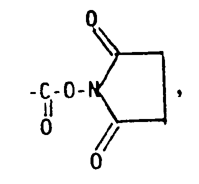 sulfate, et sulfonyle, liés à un atome de silicium ou à un atome de carbone d'un groupement hydrocarboné relié à la chaîne organohydrogénopolysiloxane par une liaison Si-C le procédé consistant à **:**
- disperser dans un solvant organique non miscible à l'eau une suspension aqueuse de charges magnétisables non revêtues d'agent dispersant, charges présentant une taille généralement inférieure à 300 10⁻⁴ µm, ainsi qu'un catalyseur d'hydrosilylation
- à dissoudre dans la phase organique de la dispersion obtenue, un mélange
. d'au moins un organopolysiloxane (appelé SiVi) de formule I suivante :
R'R₂ Si O (Si R R'' O)ₙ (Si R' R'' O)ₘ Si R₂ R' (I)
formule dans laquelle :
. les symboles R sont identiques ou différents et représentent un radical alkyle en C₁ - C₄, phényle, trifluoro -3,3,3 propyle ;
. les symboles R' sont identiques ou différents et représentent R ou un radical vinyle, le nombre de radicaux vinyles étant de 2 au moins par macromolécule ;
. les symboles R'' sont identiques ou différents et représentent R ou un motif -r-X, où r représente un radical organique bivalent et X un groupe ionogène et/ou réactif non vinylique et non polycondensable choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 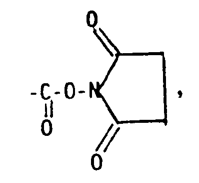 sulfate, et sulfonyle,
. au moins 60 % des radicaux représentés par les symboles R et R' sont des radicaux méthyles
. les symboles n et m peuvent être nuls séparément, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000 par molécule d'organopolysiloxane SiVi et d'organohydrogénopolysiloxane SiH
. et d'au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25° C et portant éventuellement des motifs ionogènes et/ou réactifs non vinyliques choisi parmi les groupements époxy, hydroxy, carboxy, aldéhyde, ester, acétoester, mercapto, mercaptoester, amino, alkylamino, dialkylamino, trialkylamino, ammonium quaternaire, aminoalcool, amido, hydrazide, hydrazino, halogénoalkyle en C₁-C₃,
halogénobenzyle, cyano, cyanato, 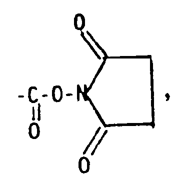 sulfate, et sulfonyle, liés à un atome de silicium ou à un atome de carbone d'un groupement hydrocarboné relié à la chaîne organohydrogénopolysiloxane par une liaison Si-C
- à réticuler le mélange de polymères SiVi et SiH
- à éliminer l'eau
- à séparer les microsphères magnétisables
- et éventuellement à redisperser lesdites microsphères dans de l'eau.

2. Procédé selon la revendication 1 caracterisé en ce que les charges magnétisables présentent une taille de 50 à 120 10⁻⁴ µm.

3. Procédé selon la revendication 1 ou 2 caractérisées en ce que la quantité de charges magnétisables constituant le coeur représente 5 à 98 % du poids des microsphères.

4. Procédé selon l'une quelconque des revendications 1 à 3 caracterisé en ce que l'organohydrogenopolysiloxane SiH a pour formule
Y R₂ Si O (R R'' SiO)ₚ (YR SiO)_{q} SiR₂ Y (IV)
formule dans laquelle les symboles R sont identiques ou différents et ont la définition donnée ci-dessus, avec au moins 80 % de radicaux méthyles, le symbole Y représente R ou un atome d'hydrogène, le nombre d'atomes d'hydrogène étant de 3 au moins par molécule de polymère, le symbole R'' a la définition donnée à la revendication 1, les symboles p et q étant tels que ledit polymère SiH présente une viscosité de l'ordre de 5 à 1500 mPas à 25°C et un nombre de motifs ionogènes et/ou réactifs non vinyliques éventuels allant de 1 à 1000, par molécule d'organohydrogénopolysiloxane SiH et d'organopolysiloxane SiVi.

5. Procédé selon l'une quelconque des revendications 1 à 4 caractérisé en ce que les quantités relatives d'organopolysiloxane SiVi et d'organohydrogenosiloxane SiH correspondent à un rapport en nombre des "groupes SiH" sur les "groupes SiVi" compris entre 0,75/1 et 4/1.

6. Procédé selon l'une des revendications 1 à 5 caractérisé en ce que les charges magnétisables ont une taille de l'ordre de 50 à 120 10⁻⁴ µm.

7. Procédé selon l'une des revendications 1 à 6 caractérisé en ce que la concentration de charges magnétisables dans la suspension aqueuse est de l'ordre de 0,5 à 50% en poids et en ce que la quantité de charges mise en oeuvre est telle que le rapport pondéral charges magnétisables/mélange de polymères SiVi et SiH soit de l'ordre de 0,005 à 50.

8. Procédé selon l'une quelconque des revendications 1 à 7 caractérisé en ce que la quantité de solvant organique mise en oeuvre est telle que le rapport pondéral phase aqueuse/phase organique soit de l'ordre de 0,005 à 2.

9. Utilisation des microsphères magnétisables obtenues selon le procédé faisant l'objet de l'une quelconque des revendications 1 à 8 comme supports actifs en biologie, à condition que les méthodes de traitement thérapeutique telles que définies à l'article 52(4) CBE soient exclues.

## Patentansprüche (Patentansprüche für folgende(n) Vertragsstaat(en): AT, BE, CH, DE, DK, FR, GB, GR, IT, LI, LU, NL, SE)

1. Magnetisierbare Mikrokugeln auf der Basis von vernetztem Organopolysiloxan, die als solche oder in wässriger Dispersion vorliegen, dadurch gekennzeichnet, daß sie gebildet sind aus:
- einem Kern, der im wesentlichen aus magnetisierbaren, nicht mit einem hydrophoben oberflächenaktiven Mittel oder einem Dispergiermittel umhüllten Füllungen besteht, die im allgemeinen eine Größe kleiner als 300 10⁻⁴ µm aufweisen
- und einer Schale auf der Basis von vernetztem Organopolysiloxan, erhalten aus der Hydrosilylierung
. von wenigstens einem Organopolysiloxan (bezeichnet als SiVi) mit der folgenden Formel I:
R'R₂ Si O (Si R R'' O)ₙ (Si R' R'' O)ₘ Si R₂ R' (I),
wobei in der Formel:
. die Symbole R gleich oder verschieden sind und einen C₁-C₄-Alkylrest, Phenyl, Trifluor-3,3,3-propyl bedeuten;
. die Symbole R' gleich oder verschieden sind und R oder einen Vinylrest bedeuten, wobei die Zahl der Vinylreste mindestens 2 pro Makromolekül beträgt;
. die Symbole R'' gleich oder verschieden sind und R oder eine Gruppe -r-X bedeuten, worin r einen zweiwertigen organischen Rest darstellt und X eine ionogene und/oder reaktive, nicht-vinylische und nicht-polykondensierbare Gruppe ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohol, Amido, Hydrazid, Hydrazino, C₁-C₃-Halogenalkyl, Halogenbenzyl, Cyano, Cyanato, 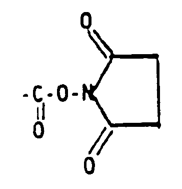 Sulfat und Sulfonyl, darstellt,
. mindestens 60% der durch die Symbole R und R' dargestellten Reste Methylreste sind;
. die Symbole n und m jeweils unabhängig voneinander Null sein können, wobei R' einen Vinylrest bedeutet, wenn m Null ist, und einen Wert haben, der ausreichend ist, um eine Viskosität des Polymeren von 20 mPas bis 30.000.000 mPas bei 25°C und eine Anzahl von iogenen und/oder reaktiven, nicht-vinylischen Gruppen von gegebenenfalls zwischen 1 bis 1000 pro Organopolysiloxan-SiVi-Molekül und Organosilan (Organohydrogenpolysiloxan) SiH, zu gewährleisten,
. mit mindestens einem Organopolysilan (Organohydrogenpolysiloxan) (bezeichnet SiH), welches pro Molekül mindestens 3 Wasserstoffatome, jedes gebunden an ein Siliciumatom, enthält, mit einer Viskosität im Bereich von 5 bis 1500 mPas bei 25°C und welches gegebenenfalls ionogene und/oder reaktive, nicht-vinylische Gruppen trägt, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohl, Amido, Hydrazid, Hydrazino,
C₁-C₃-Halogenalkyl, Halogenbenzyl, Cyano, Cyanato, 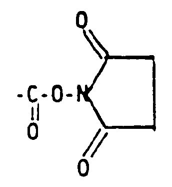 Sulfat und Sulfonyl, gebunden an ein
Siliciumatom oder ein Kohlenstoffatom einer Kohlenwasserstoffgruppe, wiederum über eine Si-C-Bindung gebunden an eine Organosilankette (Organohydrogenpolysiloxankette).

2. Mikrokugeln nach Anspruch 1, dadurch gekennzeichnet, daß die magnetisierbaren Füllungen eine Größe von 5 bis 120 10⁻⁴ µm besitzen.

3. Mikrokugeln nach Anspruch 1 oder 2, dadurch gekennzeichnet, daß die Menge der magnetisierbaren Füllungen, die den Kern bilden, 5 bis 98 Gew.% der Mikrokugeln darstellt.

4. Mikrokugeln nach einem der Ansprüche 1 bis 3, dadurch gekennzeichnet, daß das Organopolysilan (Organohydrogenpolysiloxan) SiH eine Formel
Y R₂ Si O (R R'' SiO)ₚ (YR SiO)_{q} SiR₂ Y
hat, wobei in der Formel die Symbole R gleich oder verschieden sind und wie oben definiert sind, mit mindestens 80% Methylresten, das Symbol Y R oder ein Wasserstoffatom bedeutet, wobei die Zahl der Wasserstoffatome mindestens 3 pro Polymermolekül beträgt, das Symbol R'' wie in Anspruch 1 definiert ist, die Symbole p und q solche sind, daß das genannte Polymere SiH eine Viskosität im Bereich von 5 bis 1500 mPas bei 25°C hat und eine Zahl von ionogenen und/oder reaktiven, nicht-vinylischen Gruppen gegebenenfalls von 1 bis 1000 pro Organopolysilanmolekül (Organohydrogenpolysiloxan) SiH und Organopolysiloxan SiVi reicht.

5. Mikrokugeln nach einem der Ansprüche 1 bis 4, dadurch gekennzeichnet, daß die relativen Mengen von Organopolysiloxan SiVi und Organopolysilan (Organohydrogenpolysiloxan) SiH einem Zahlenverhältnis der "Gruppen SiH" zu den "Gruppen SiVi" zwischen 0,75/1 und 4/1 entsprechen.

6. Verfahren zur Herstellung von magnetisierbaren Mikrokugeln auf der Basis von vernetztem Organopolysiloxan, bestehend aus:
- Dispergieren einer wässrigen Suspension der magnetisierbaren, nicht mit einem Dispergiermittel umhüllten Füllungen, wobei die Füllungen im allgemeinen eine Größe kleiner als 300 10⁻⁴ µm aufweisen, sowie eines Hydrosilylierkatalysators in einem organischen, mit Wasser nicht mischbaren Lösungsmittel,
- Auflösen eines Gemisches aus mindestens einem Organopolysiloxan (bezeichnet SiVi) mit der folgenden Formel I
R'R₂ Si O (Si R R'' O)ₙ (Si R' R'' O)ₘ Si R₂ R' (I)
wobei in der Formel:
. die Symbole R gleich oder verschieden sind und einen C₁-C₄-Alkylrest, Phenyl, Trifluor-3,3,3-propyl bedeuten;
. die Symbole R' gleich oder verschieden sind und R oder einen Vinylrest bedeuten, wobei die Zahl der Vinylreste mindestens 2 pro Makromolekül ist;
. die Symbole R'' gleich oder verschieden sind und R oder eine Gruppe -r-X bedeuten, worin r einen zweiwertigen organischen Rest darstellt und X eine ionogene und/oder reaktive, nicht-vinylische und nicht-polykondensierbare Gruppe, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohol, Amido, Hydrazid, Hydrazino, C₁-C₃-Halogenalkyl, Halogenbenzyl, Cyano, Cyanato, 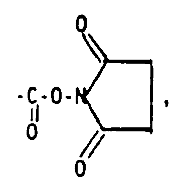 Sulfat und Sulfonyl, darstellt;
. mindestens 60% der durch die Symbole R und R' dargestellten Reste Methylreste sind;
. die Symbole n und m jeweils unabhängig voneinander Null sein können, wobei R' einen Vinylrest bedeutet, wenn m Null ist, und einen Wert haben, der ausreichend ist, um eine Viskosität des Polymeren von 20 mPas bis 30.000.000 mPas bei 25°C und eine Anzahl von iogenen und/oder reaktiven, nicht-vinylischen Gruppen, von gebenenfalls zwischen 1 bis 1000 pro Organopolysiloxanmolekül SiVi und Organosilanmolekül (Organohydroxygenpolysiloxanmolekül) SiH, zur Verfügung zu stellen,
- und aus mindestens einem Organopolysilan (Organohydrogenpolysiloxan) (bezeichnet SiH), welches pro Molekül mindestens 3 Wasserstoffatome, jedes gebunden an ein Siliciumatom, enthält, mit einer Viskosität im Bereich von 5 bis 1500 mPas bei 25°C, und welches gegebenenfalls ionogene und/oder reaktive, nicht-vinylische Gruppen trägt, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohl, Amido, Hydrazid, Hydrazino,
C₁-C₃-Halogenalkyl, Halogenbenzyl, Cyano, Cyanato, 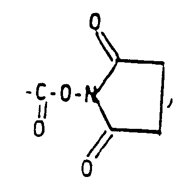 Sulfat und Sulfonyl, gebunden an ein Siliciumatom oder ein Kohlenstoffatom einer Kohlenwasserstoffgruppe, wiederum über eine Si-C-Bindung gebunden an eine Organosilankette, in der organischen Phase der erhaltenen Dispersion,
- Vernetzen des Gemisches aus den Polymeren SiVi und SiH
- Entfernen des Wassers
- Abtrennen der magnetisierbaren Mikrokugeln
- und gegebenenfalls erneutem Dispergieren der genannten Mikrokugeln in Wasser.

7. Verfahren nach Anspruch 6, dadurch gekennzeichnet, daß die magnetisierbaren Füllungen eine Größe im Bereich von 50 bis 120 10⁻⁴ µm aufweisen.

8. Verfahren nach einem der Ansprüche 6 oder 7, dadurch gekennzeichnet, daß die Konzentration der magnetisierbaren Füllungen in der wässrigen Suspension im Bereich von 0,5 bis 50 Gew.% liegt und daß die Menge der verwendeten Füllungen so ist, daß das Gewichtsverhältnis der magnetisierbaren Füllungen/Polymergemisch SiVi und SiH in dem Bereich von 0,005 bis 50 liegt.

9. Verfahren nach einem der Ansprüche 6 bis 8, dadurch gekennzeichnet, daß die Menge des verwendeten organischen Lösungsmittels so ist, daß das Gewichtsverhältnis wässrige Phase/organische Phase in dem Bereich von 0,005 bis 2 liegt.

10. Verfahren nach einem der Ansprüche 6 bis 9, dadurch gekennzeichnet, daß das Organopolysilan (Organohydrogenpolysiloxan) die Formel
Y R₂ Si O (R R'' SiO)ₚ (YR SiO)_{q} SiR₂ Y
hat, wobei in der Formel die Symbole R gleich oder verschieden sind und wie oben definiert sind, mit mindestens 80% Methylresten, das Symbol Y R oder ein Wasserstoffatom bedeutet, wobei die Zahl der Wasserstoffatome wenigstens 3 pro Polymermolekül ist, das Symbol R'' wie in Anspruch 6 definiert ist, wobei die Symbole p und q solche sind, daß das genannte Polymere SiH eine Viskosität im Bereich von 5 bis 1500 mPas bei 25°C hat und daß eine Zahl von ionogenen und/oder reaktiven, nicht-vinylischen Gruppen gegebenenfalls von 1 bis 1000 pro Organopolysilanmolekül (Organohydrogenpolysiloxanmolekül) SiH und Organopolysiloxanmolekül SiVi reicht.

11. Verfahren nach einem der Ansprüche 6 bis 10, dadurch gekennzeichnet, daß die relativen Mengen von Organopolysiloxan SiVi und Organopolysilan (Organohydrogenpolysiloxan) SiH einem Zahlenverhältnis der Gruppen SiH" zu den "Gruppen SiVi" zwischen 0,75/1 und 4/1 entsprechen.

12. Verwendung der magnetisierbaren Mikrokugeln gemäß einem der Ansprüche 1 bis 5 oder erhalten nach dem Verfahren, welches Gegenstand eines der Ansprüche 6 bis 11 ist, als aktive biologische Träger, mit der Maßgabe, daß die in Artikel 52(4) EPÜ definierten therapeutischen Behandlungsmethoden ausgeschlossen sind.

## Patentansprüche (Patentansprüche für folgende(n) Vertragsstaat(en): ES)

1. Verfahren zur Herstellung von magnetisierbaren Mikrokugeln auf der Basis von vernetztem Organopolysiloxan, die als solche oder in wässriger Suspension vorliegen, dadurch gekennzeichnet, daß sie gebildet sind aus:
- einem Kern, der im wesentlichen aus magnetisierbaren, nicht mit einem hydrophoben oberflächenaktive Mittel oder einem Dispergiermittel umhüllten Füllungen besteht, die im allgemeinen eine Größe kleiner als 300 10⁻⁴ µm aufweisen
- und einer Schale auf der Basis von vernetztem Organopolysiloxan,
erhalten aus der Hydrosilylierung
- von mindestens einem Organopolysiloxan (bezeichnet SiVi) mit der folgenden Formel I:
R'R₂ Si O (Si R R'' O)ₙ (Si R' R'' O)ₘ Si R₂ R' (I)
wobei in der Formel:
. die Symbole R gleich oder verschieden sind und einen C₁-C₄-Alkylrest, Phenyl, Trifluor-3,3,3-propyl bedeuten;
. die Symbole R' gleich oder verschieden sind und R oder einen Vinylrest bedeuten, wobei die Zahl der Vinylreste mindestens 2 pro Makromolekül beträgt;
. die Symbole R'' gleich oder verschieden sind und R oder eine Gruppe -r-X- bedeuten, worin r einen zweiwertigen organischen Rest darstellt und X eine ionogene und/oder reaktive, nicht-vinylische und nicht-polykondensierbare Gruppe ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohol, Amido, Hydrazid, Hydrazino, C₁-C₃-Halogenalkyl,
Halogenbenzyl, Cyano, Cyanato, 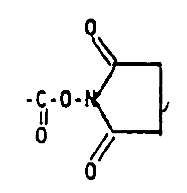 Sulfat und Sulfonyl darstellt,
. mindestens 60% der durch die Symbole R und R' dargestellten Reste Methylreste sind;
. die Symbole n und m jeweils unabhängig voneinenader Null sein können, wobei R' einen Vinylrest bedeutet, wenn m Null ist, und einen Wert haben, der ausreichend ist, um eine Viskosität des Polymeren von 20 mPas bis 30.000.000 mPas bei 25°C und eine Anzahl von iogenen und/oder reaktiven, nicht-vinylischen Gruppen von gegebenenfalls zwischen 1 bis 1000 pro Organopolysiloxanmolekül SiVi und Organosilanmolekül (Organohydrogenpolysiloxan) SiH zur Verfügung zu stellen,
- mit mindestens einem Organopolysilan (Organohydrogenpolysiloxan)(bezeichnet SiH), welches pro Molekül mindestens 3 Wasserstoffatome, jedes gebunden an ein Siliciumatom, enthält, mit einer Viskosität im Bereich von 5 bis 1500 mPas bei 25°C, und welches gegebenenfalls ionogene und/oder reaktive, nicht-vinylische Gruppen trägt, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohl, Amido, Hydrazid, Hydrazino,
C₁-C₃-Halogenalkyl, Halogenbenzyl, Cyano, Cyanato, 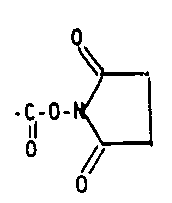 Sulfat und Sulfonyl, gebunden an ein Siliciumatom oder ein Kohlenstoffatom einer Kohlenwasserstoffgruppe, wiederum über eine Si-C-Bindung gebunden an eine Organopolysilankette (Organohydrogenpolysiloxankette), wobei das Verfahren besteht aus:
- Dispergieren einer wässrigen Suspension der magnetisierbaren, nicht mit einem Dispergiermittel umhüllten Füllungen, wobei die Füllungen im allgemeinen eine Größe kleiner als 300 10⁻⁴ µm aufweisen, sowie eines Hydrosilylierkatalysators in einem organischen, mit Wasser nicht mischbaren Lösungsmittel,
- Auflösen eines Gemisches aus mindestens einem Organopolysiloxan (bezeichnet SiVi) mit der folgenden Formel I
R'R₂ Si O (Si R R'' O)ₙ (Si R' R'' O)ₘ Si R₂ R' (I)
wobei in der Formel:
. die Symbole R gleich oder verschieden sind und einen C₁-C₄-Alkylrest, Phenyl, Trifluor-3,3,3-propyl bedeuten;
. die Symbole R' gleich oder verschieden sind und R oder einen Vinylrest bedeuten, wobei die Zahl der Vinylreste 2 oder weniger pro Makromolekül beträgt;
. die Symbole R'' gleich oder verschieden sind und R oder eine Gruppe -r-X bedeuten, worin r einen zweiwertigen organischen Rest darstellt und X eine ionogene und/oder reaktive, nicht-vinylische und nicht-polykondensierbare Gruppe, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohol, Amido, Hydrazid, Hydrazino, C₁-C₃-Halogenalkyl,
Halogenbenzyl, Cyano, Cyanato, 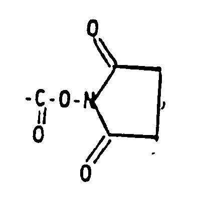 Sulfat und Sulfonyl, darstellt;
. mindestens 60% der durch die Symbole R und R' dargestellten Reste Methylreste sind;
. die Symbole n und m jeweils unabhängig voneinander Null sein können, wobei R' einen Vinylrest bedeutet, wenn m Null ist, und einen Wert haben, der ausreichend ist, um eine Viskosität des Polymeren von 20 mPas bis 30.000.000 mPas bei 25°C und eine Zahl von ionogenen und/oder reaktiven, nicht-vinylischen Gruppen, von gegebenenfalls zwischen 1 bis 1000 pro Organopolysiloxanmolekül SiVi und Organosilanmolekül (Organohydrogenpolysiloxanmolekül) SiH zur Verfügung zu stellen,
mit mindestens einem Organosilan (Organohydrogenpolysiloxan) (bezeichnet SiH), welches pro Molekül mindestens 3 Wasserstoffatome, jedes gebunden an ein Siliciumatom, enthält, mit einer Viskosität in dem Bereich von 5 bis 1500 mPas bei 25°C, und welches gegebenenfalls ionogene und/oder reaktive, nicht-vinylische Gruppen trägt, ausgewählt aus den Gruppen Epoxy, Hydroxy, Carboxy, Aldehyd, Ester, Acetoester, Mercapto, Mercaptoester, Amino, Alkylamino, Dialkylamino, Trialkylamino, quaternäres Ammonium, Aminoalkohl, Amido, Hydrazid, Hydrazino,
C₁-C₃-Halogenalkyl, Halogenbenzyl, Cyano, Cyanato, 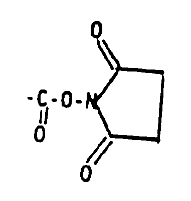 Sulfat und Sulfonyl, gebunden an ein Siliziumatom oder ein Kohlenstoffatom einer Kohlenwasserstoffgruppe, wiederum über eine Si-C-Bindung gebunden an eine Organopolysilankette (Organohydrogenpolysiloxankette) in der organischen Phase der wässrigen Dispersion,
- Vernetzen des Gemisches aus den Polymeren SiVi und SiH
- Entfernen des Wassers
- Abtrennen der magnetisierbaren Mikrokugeln
- und gegebenenfalls erneutes Dispergieren der Mikrokugeln in Wasser.

2. Verfahren nach Anspruch 1, dadurch gekennzeichnet, daß die magnetisierbaren Füllungen eine Größe von 5 bis 120 10⁻⁴ µm besitzen.

3. Verfahren nach Anspruch 1 oder 2, dadurch gekennzeichnet, daß die Menge der magnetisierbaren Füllungen, die den Kern bilden, 5 bis 98 Gew.% der Mikrokugeln darstellt.

4. Verfahren nach einem der Ansprüche 1 bis 3, dadurch gekennzeichnet, daß das Organopolysilan (Organohydrogenpolysiloxan) SiH eine Formel
Y R₂ Si O (R R'' SiO)ₚ (YR SiO)_{q} SiR₂ Y
hat, wobei in der Formel die Symbole R gleich oder verschieden sind und wie oben definiert sind, mit mindestens 80% Methylresten, das Symbol Y R oder ein Wasserstoffatom bedeutet, wobei die Zahl der Wasserstoffatome wenigstens 3 pro Polymermolekül ist, das Symbol R'' wie in Anspruch 1 definiert ist, wobei die Symbole p und q solche sind, daß das genannte Polymere SiH eine Viskosität im Bereich von 5 bis 1500 mPas bei 25°C hat und daß eine Zahl von ionogenen und/oder reaktiven, nicht-vinylischen Gruppen gegebenenfalls von 1 bis 1000 pro Organopolysilanmolekül (Organohydrogenpolysiloxanmolekül) SiH und Organopolysiloxan SiVi reicht.

5. Verfahren nach einem der Ansprüche 1 bis 4, dadurch gekennzeich-net, daß die relativen Mengen von Organopolysiloxan SiVi und Organopolysilan (Organohydrogenpolysiloxan) SiH einem Zahlenverhältnis der "Gruppen SiH" zu den "Gruppen SiVi" zwischen 0,75/1 und 4/1 entsprechen.

6. Verfahren nach einem der Ansprüche 1-5, dadurch gekennzeichnet, daß die magnetisierbaren Füllungen eine Größe in dem Bereich von 50 bis 120 10⁻⁴ µm aufweisen.

7. Verfahren nach einem der Ansprüche 6 oder 7, dadurch gekennzeichnet, daß die Konzentration der magnetisierbaren Füllungen in der wässrigen Suspension im Bereich von 0,5 bis 50 Gew.% liegt und daß die Menge der verwendeten Füllungen so ist, daß das Gewichtsverhältnis der magnetisierbaren Füllungen/Polymergemisch SiVi und SiH im Bereich von 0,005 bis 50 liegt.

8. Verfahren nach einem der Ansprüche 1 bis 7, dadurch gekennzeichnet, daß die Menge des verwendeten organischen Lösungsmittels so ist, daß das Gewichtsverhältnis wässrige Phase/organische Phase im Bereich von 0,005 bis 2 liegt.

9. Verwendung der nach dem Verfahren, welches Gegenstand eines der Ansprüche 1-8 ist, erhaltenen magnetisierbaren Mikrokugeln als biologisch aktive Träger, mit der Maßgabe, daß die in Artikel 52 (4) EPÜ definierten therapeutischen Behandlungsmethoden ausgeschlossen sind.

## Claims (Claims for the following Contracting State(s): AT, BE, CH, DE, DK, FR, GB, GR, IT, LI, LU, NL, SE)

1. Magnetizable microspheres based on crosslinked organopolysiloxane, occurring as such or in aqueous dispersion, characterized in that they are formed from:
- a core essentially consisting of magnetizable fillers not coated with a hydrophobic surfaltant or dispersing agent having a size generally smaller than 300 10⁻⁴ µm,
- and a shell based on a crosslinked organopolysiloxane derived from hydrosilylation
. of at least one organopolysiloxane (termed SiVi) of the following formula I:
R'R₂ Si O (Si R R'' O)ₙ (Si R' R'' O)ₘ Si R₂ R' (I)
in which formula:
. the symbols R are identical or different and represent a C₁-C₄ alkyl, phenyl or 3,3,3-trifluoropropyl radical;
. the symbols R' are identical or different and represent R or a vinyl radical, the number of vinyl radicals being at least 2 per macromolecule;
. the symbols R'' are identical or different and represent R or an -r-X unit, where r represents a divalent organic radical and X represents a non-vinyl and non-polycondensable ionogenic and/or reactive group, chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 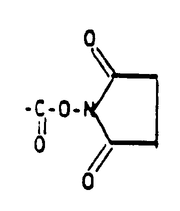 sulphate and sulphonyl groups
. at least 60 % of the radicals represented by the symbols R and R' are methyl radicals,
. the symbols n and m may separately be zero, R' representing a vinyl radical if m is zero, and have a value sufficient to ensure a viscosity of the polymer of 20 mPas to 30,000,000 mPas at 25°C and a number of optional non-vinyl ionogenic and/or reactive units ranging from 1 to 1,000 per molecule of organopolysiloxane SiVi and of organohydrogenopolysiloxane SiH,
. with at least one organohydrogenopolysiloxane (termed SiH) containing, per molecule, at least three hydrogen atoms, each bonded to a silicon atom, having a viscosity of the order of 5 to 1,500 mPas at 25°C, and optionally carrying non-vinyl ionogenic and/or reactive units chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 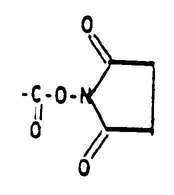 sulfate and sulphonyl groups bonded to a silicon atom or to a carbon atom of a hydrocarbon group linked to the organohydrogenopolysiloxane chain by a Si-C bond.

2. Microspheres according to claim 1, characterized in that the magnetizable fillers have a size of from 50 to 120 10⁻⁴ µm.

3. Microspheres according to claim 1 or 2, characterized in that the amount of magnetizable fillers making up the core represents 5 to 98 % by weight of the microspheres.

4. Microspheres according to any one of claims 1 to 3, characterized in that the organohydrogenopolysiloxane SiH has the formula
Y R₂ Si O (R R'' SiO)ₚ (YR SiO)_{q} SiR₂ Y (IV)
in which formula the symbols R are identical or different and have the definition given above, with at least 80 % of methyl radicals, the symbol Y represents R or a hydrogen atom, the number of hydrogen atoms being at least 3 per molecule of polymer, the symbol R'' has the definition given in claim 1, the symbols p and q being such that the said polymer SiH has a viscosity of the order of 5 to 1,500 mPas at 25°C and a number of optional non-vinyl ionogenic and/or reactive units ranging from 1 to 1,000 per molecule of organohydrogenopolysiloxane SiH and of organopolysiloxane SiVi.

5. Microspheres according to any one of claims 1 to 4, characterized in that the relative amounts of organopolysiloxane SiVi and organohydrogenosiloxane SiH correspond to a number-ratio of "SiH groups" to "SiVi groups" of between 0.75/1 and 4/1.

6. Process for the preparation of magnetizable microspheres based on crosslinked organopolysiloxane, consisting in:
- dispersing an aqueous suspension of magnetizable fillers not coated with dispersing agent, said fillers having a size generally smaller than 300 10⁻⁴ µm and also a hydrosilylation catalyst, in a water-immiscible organic solvent,
- dissolving a mixture of
. at least one organopolysiloxane (termed SiVi) of formula I below:
R'R₂ Si O (Si R R'' O)ₙ (Si R' R'' O)ₘ Si R₂ R' (I)
in which formula:
. the symbols R are identical or different and represent a C₁-C₄ alkyl, phenyl or 3,3,3-trifluoropropyl radical;
. the symbols R' are identical or different and represent R or a vinyl radical, the number of vinyl radicals being at least 2 per macromolecule;
. the symbols R'' are identical or different and represent R or an -r-X unit, where r represents a divalent organic radical and X represents a non-vinyl and non-polycondensable ionogenic and/or reactive group chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 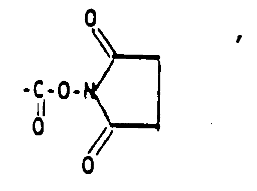 sulphate and sulphonyl groups
. at least 60 % of the radicals represented by the symbols R and R' are methyl radicals,
. the symbols n and m may separately be zero, R' representing a vinyl radical if m is zero, and have a value sufficient to ensure a viscosity of the polymer of 20 mPas to 30,000,000 mPas at 25°C and a number of optional non-vinyl ionogenic and/or reactive units ranging from 1 to 1,000 per molecule of organopolysiloxane SiVi and of organohydrogenopolysiloxane SiH,
. and at least one organohydrogenopolysiloxane (termed SiH) containing, per molecule, at least three hydrogen atoms each linked to a silicon atom, having a viscosity of the order of 5 to 1,500 mPas at 25°C and optionally carrying non-vinyl ionogenic and/or reactive units chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 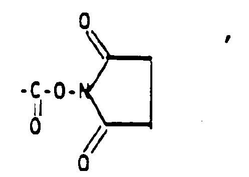 sulphate and sulphonyl groups bonded to a silicon atom or to a carbon atom of a hydrocarbon group linked to the organohydrogenopolysiloxane chain by a Si-C bond, in the organic phase of the dispersion obtained,
- crosslinking the mixture of polymers SiVi and SiH,
- removing the water,
- separating off the magnetizable microspheres
- and, if appropriate, redispersing the said microspheres in water.

7. Process according to claim 6, characterized in that the magnetizable fillers have a size of the order of 50 to 120 10⁻⁴ µm.

8. Process according to claim 6 or 7, characterized in that the concentration of magnetizable fillers in the aqueous suspension is of the order of 0.5 to 50 % by weight and in that the amount of fillers used is such that the ratio by weight of magnetizable fillers/mixture of polymers SiVi and SiH is of the order of 0.005 to 50.

9. Process according to any one of claims 6 to 8, characterized in that the amount of organic solvent used is such that the ratio by weight of aqueous phase/organic phase is of the order of 0.005 to 2.

10. Process according to any one of claims 6 to 9, characterized in that the organohydrogenopolysiloxane has the formula
Y R₂ Si O (R R'' SiO)ₚ (YR SiO)_{q} SiR₂ Y (IV)
in which formula the symbols R are identical or different and have the definition given above, with at least 80 % of methyl radicals, the symbol Y represents R or a hydrogen atom, the number of hydrogen atoms being at least 3 per molecule of polymer, the symbol R'' has the definition given in claim 6, the symbols p and q being such that the said polymer SiH has a viscosity of the order of 5 to 1,500 mPas at 25°C and a number of optional non-vinyl ionogenic and/or reactive units ranging from 1 to 1,000 per molecule of organohydrogenopolysiloxane SiH and of organopolysiloxane SiVi.

11. Process according to any one of claims 6 to 10, characterized in that the relative amounts of organopolysiloxane SiVi and organohydrogenosiloxane SiH correspond to a number-ratio of "SiH groups" to the "SiVi groups" of between 0.75/1 and 4/1.

12. Use of the magnetizable microspheres which are the subject of any one of claims 1 to 5 or obtained according to the process which is the subject of any one of claims 6 to 11 as active supports in biology, proviso that the therapeutic method of treatment such as defined at article 52 (4) EPC be disclaimed.

## Claims (Claims for the following Contracting State(s): ES)

1. Process of preparation of magnetizable microspheres based on crosslinked organopolysiloxane, occuring as such or in aqueous dispersion, characterized in that they are formed from:
- a core essentially consisting of magnetizable fillers not coated with a hydrophobic surfactant or dispersing agent having a size generally smaller than 300 10⁻⁴ µm,
- and a shell based on a crosslinked organopolysiloxane derived from hydrosilylation
. of at least one organopolysiloxane (termed SiVi) of the following formula I:
R'R₂ Si O (Si R R'' O)ₙ (Si R' R'' O)ₘ Si R₂ R' (I)
in which formula:
. the symbols R are identical or different and represent a C₁-C₄ alkyl, phenyl or 3,3,3-trifluoropropyl radical;
. the symbols R' are identical or different and represent R or a vinyl radical, the number of vinyl radicals being at least 2 per macromolecule;
. the symbols R'' are identical or different and represent R or an -r-X unit, where r represents a divalent organic radical and X represents a non-vinyl and non-polycondensable ionogenic and/or reactive group chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 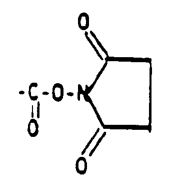 sulphate and sulphonyl groups
. at least 60 % of the radicals represented by the symbols R and R' are methyl radicals,
. the symbols n and m may separately be zero, R' representing a vinyl radical if m is zero, and have a value sufficient to ensure a viscosity of the polymer of 20 mPas to 30,000,000 mPas at 25°C and a number of optional non-vinyl ionogenic and/or reactive units ranging from 1 to 1,000 per molecule of organopolysiloxane SiVi and of organohydrogenopolysiloxane SiH,
. with at least one organohydrogenopolysiloxane (termed SiH) containing, per molecule, at least three hydrogen atoms, each bonded to a silicon atom, having a viscosity of the order of 5 to 1,500 mPas at 25°C, and optionally carrying non-vinyl ionogenic and/or reactive units chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 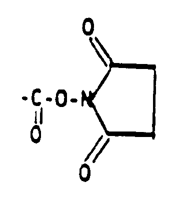 sulphate and sulphony groups bonded to a silicon atom or to a carbon atom of a hydrocarbon group linked to the organohydrogenopolysiloxane chain by a Si-C bond said process consisting in:
- dispersing an aqueous suspension of magnetizable fillers not coated with dispersing agent, said fillers having a size generally smaller than 300 10⁻⁴ µm and also a hydrosilylation catalyst, in a water-immiscible organic solvent,
- dissolving in the organic phase of the dispersion obtained a mixture of
. at least one organopolysiloxane (termed SiVi) of formula I below:
R'R₂ Si O(Si R R'' O)ₙ (Si R' R'' O)ₘ Si R₂ R' (I)
in which formula:
. the symbols R are identical or different and represent a C₁-C₄ alkyl, phenyl or 3,3,3-trifluoropropyl radical;
. the symbols R' are identical or different and represent R or a vinyl radical, the number of vinyl radicals being at least 2 per macromolecule;
. the symbols R'' are identical or different and represent R or an -r-X unit, where r represents a divalent organic radical and X represents a non-vinyl and non-polycondensable ionogenic and/or reactive group chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 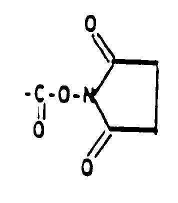 sulphate and sulphonyl groups
. at least 60 % of the radicals represented by the symbols R and R' are methyl radicals,
. the symbols n and m may separately be zero, R' representing a vinyl radical if m is zero, and have a value sufficient to ensure a viscosity of the polymer of 20 mPas to 30,000,000 mPas at 25°C and a number of optional non-vinyl ionogenic and/or reactive units ranging from 1 to 1,000 per molecule of organopolysiloxane SiVi and of organohydrogenopolysiloxane SiH,
. and at least one organohydrogenopolysiloxane (termed SiH) containing, per molecule, at least three hydrogen atoms each linked to a silicon atom, having a viscosity of the order of 5 to 1,500 mPas at 25°C and optionally carrying non-vinyl ionogenic and/or reactive units chosen among the epoxy, hydroxyl, carboxyl, aldehyde, ester, acetoester, mercapto, mercaptoester, amino, alkylamino, dialkylamino, trialkylamino, quaternary ammonium, aminoalcohol, amido, hydrazide, hydrazino, C₁-C₃ haloalkyl, halobenzyl, cyano, cyanato, 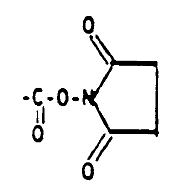 sulphate and sulphonyl groups bonded to a silicon atom or to a carbon atom of a hydrocarbon group linked to the organohydrogenopolysiloxane chain by a Si-C bond,
- crosslinking the mixture of polymers SiVi and SiH,
- removing the water,
- separating off the magnetizable microspheres
- and, if appropriate, redispersing the said microspheres in water.

2. Process according to claim 1, characterized in that the magnetizable fillers have a size of from 50 to 120 10⁻⁴ µm.

3. Process according to claim 1 or 2, characterized in that the amount of magnetizable fillers making up the core represents 5 to 98 % by weight of the microspheres.

4. Process according to any one of claims 1 to 3, characterized in that the organohydrogenopolysiloxane SiH has the formula
Y R₂ Si O (R R'' SiO)ₚ (YR SiO)_{q} SiR₂ Y (IV)
in which formula the symbols R are identical or different and have the definition given above, with at least 80 % of methyl radicals, the symbol Y represents R or a hydrogen atom, the number of hydrogen atoms being at least 3 per molecule of polymer, the symbol R'' has the definition given in claim 1, the symbols p and q being such that the said polymer SiH has a viscosity of the order of 5 to 1,500 mPas at 25°C and a number of optional non-vinyl ionogenic and/or reactive units ranging from 1 to 1,000 per molecule of organohydrogenopolysiloxane SiH and of organopolysiloxane SiVi.

5. Process according to any one of claims 1 to 4, characterized in that the relative amounts of organopolysiloxane SiVi and organohydrogenosiloxane SiH correspond to a number-ratio of "SiH groups" to "SiVi groups" of between 0.75/1 and 4/1.

6. Process according to any of claims 1 to 5, characterized in that the magnetizable fillers have a size of the order of 50 to 120 10⁻⁴ µm.

7. Process according to any one of claims 1 to 6, characterized in that the concentration of magnetizable fillers in the aqueous suspension is of the order of 0.5 to 50 % by weight and in that the amount of fillers used is such that the ratio by weight of magnetizable fillers/mixture of polymers SiVi and SiH is of the order of 0.005 to 50.

8. Process according to any one of claims 1 to 7, characterized in that the amount of organic solvent used is such that the ratio by weight of aqueous phase/organic phase is of the order of 0.005 to 2.

9. Use of the magnetizable microspheres obtained according to the process which is the subject of any one of claims 1 to 8 as active supports in biology, proviso that the therapeutic methods of treatment such as defined at article 52(4) be disclaimed.
